(19) Europäisches Patentamt
European Patent Office
Office européen des brevets

(11) Publication number: **0 350 950 A1**

# (12) EUROPEAN PATENT APPLICATION

(21) Application number: 89112960.3

(22) Date of filing: 14.07.89

(51) Int. Cl.4: C07D 215/56 , C07D 401/04 , C07D 471/04 , //(C07D471/04, 221:00,221:00)

(30) Priority: 15.07.88 US 219673

(43) Date of publication of application: 17.01.90 Bulletin 90/03

(84) Designated Contracting States: ES GR

(71) Applicant: ABBOTT LABORATORIES
One Abbott Park Road
Abbott Park, IL 60064-3500(US)

(72) Inventor: Faubl, Hermann
511 Lincoln Avenue
Lake Bluff Illinois 60044(US)
Inventor: Chu, Daniel T.
204 Ashland Avenue
Vernon Hills Illinois 60061(US)

(74) Representative: Modiano, Guido et al
MODIANO, JOSIF, PISANTY & STAUB
Modiano & Associati Via Meravigli, 16
I-20123 Milano(IT)

## (54) Process for the preparation of quinoline antibacterial compounds.

(57) A process for preparing substituted 7-(piperazin-1-yl)-4-oxo-1,4-dihydro-3-quinoline carboxylic acid derivative antibacterial agents by reacting a carbamate protected piperazine derivative with a 7-halo-4-oxo-1,4-dihydroquinoline carboxylic acid derivative.

EP 0 350 950 A1

# PROCESS FOR THE PREPARATION OF QUINOLINE ANTIBACTERIAL COMPOUNDS

## Technical Field

This invention relates to a new process for preparing quinoline antibacterial compounds.

## Background Art

It is known that certain 7-(piperazin-1-yl)-4-oxo-1,4-dihydroquinoline-3-carboxylic acid derivatives exhibit antibacterial activity. These compounds and a method for their preparation are disclosed in U.S. Patent No. 4,730,000, issued March 8, 1988, which is hereby incorporated by reference. In particular, the process discloses the reaction of a 7-chloro-1-(halo-substituted phenyl)-6-fluoro-1,4-dihydro-4-oxo-quinoline-3-carboxylic acid (1, $R_1$ = halogen, $R_2$ = hydrogen or halogen) with an unsubstituted or substituted piperazine (2, $R_3$ = hydrogen or loweralkyl) to provide the desired antibacterial quinoline (3).

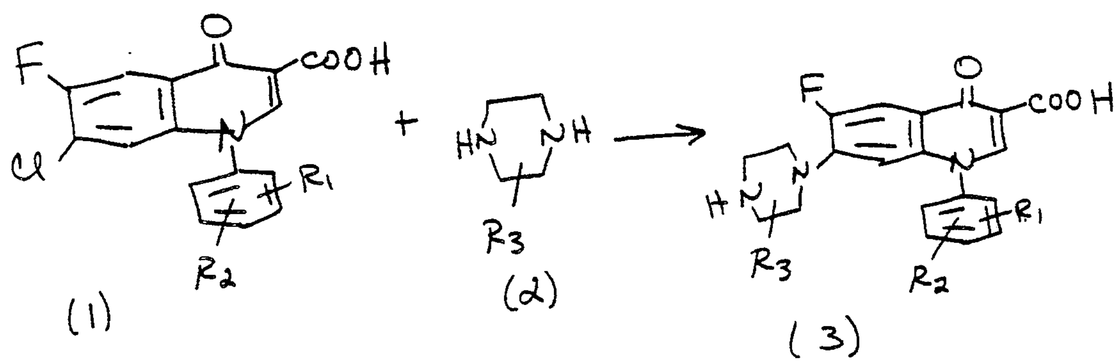

(1) (2) (3)

This process provides (3) along with a certain amount of a mixture of (3) and a contaminating compound thought to result from the reaction of the piperazine with the 1-(halo-substituted phenyl) substitutent. This mixture is difficult to separate and thus it is difficult to obtain optimum yields of pure (3).

## Disclosure of the Invention

The present invention relates to a process for preparing substituted 7-(piperazin-1-yl)-4-oxo-1,4-dihydro-3-quinoline carboxylic acid derivative antibacterial agents which provides the quinoline antibacterial agent in a high state of purity.

In particular, the process involves the reaction of a solution of an N-protected (carbamate) derivative of a piperazine (4, $R_3$ = hydrogen, loweralkyl, haloalkyl, loweralkenyl, loweralkynyl, $C_3$ to $C_7$ cycloalkyl, hydroxyalkyl, alkoxyalkyl, (N-protected)(alkyl)aminoalkyl, dialkylaminoalkyl, (N-protected)aminoalkyl, hydroxy, alkoxy, halogen, alkanoyl, alkanoylamino, (N-protected)amino, (N-protected)(alkyl)amino or dialkylamino; $R_4$ = loweralkyl or benzyl) with substituted quinoline (1, R = hydrogen or a carboxy-protecting group; $R_7$ = loweralkyl, loweralkenyl, $C_3$ to $C_7$ cycloalkyl, haloalkyl or

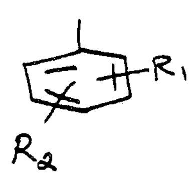

wherein $R_1$ is halogen and $R_2$ is hydrogen or halogen; X = CH, CCl, CF or N; and Y = F or Cl) in the presence of a base. The resulting carbamate (5) can be isolated in highly pure form and then the carbamate is hydrolyzed under acidic conditions ($R_4$ = loweralkyl or benzyl) or hydrogenolyzed ($R_4$ = benzyl), and, optionally, deprotected when R is a carboxy-protecting group, to provide quinoline (3, R = H).

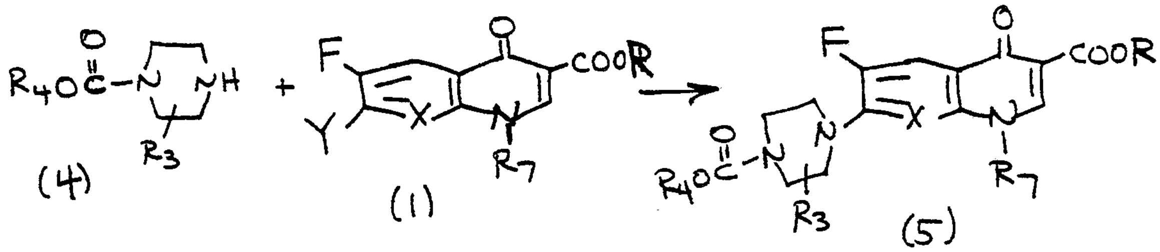

(4) (1) (5)

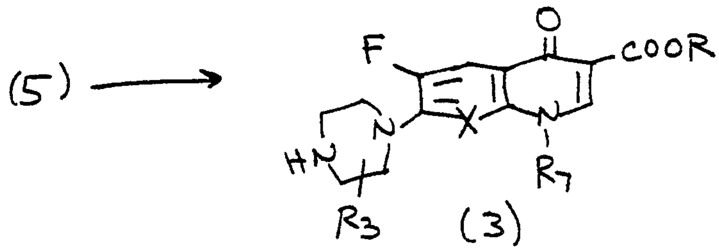

(5) (3)

The N-protected (carbamate) piperazine derivatives (4) are prepared according to Scheme 1.

Scheme 1

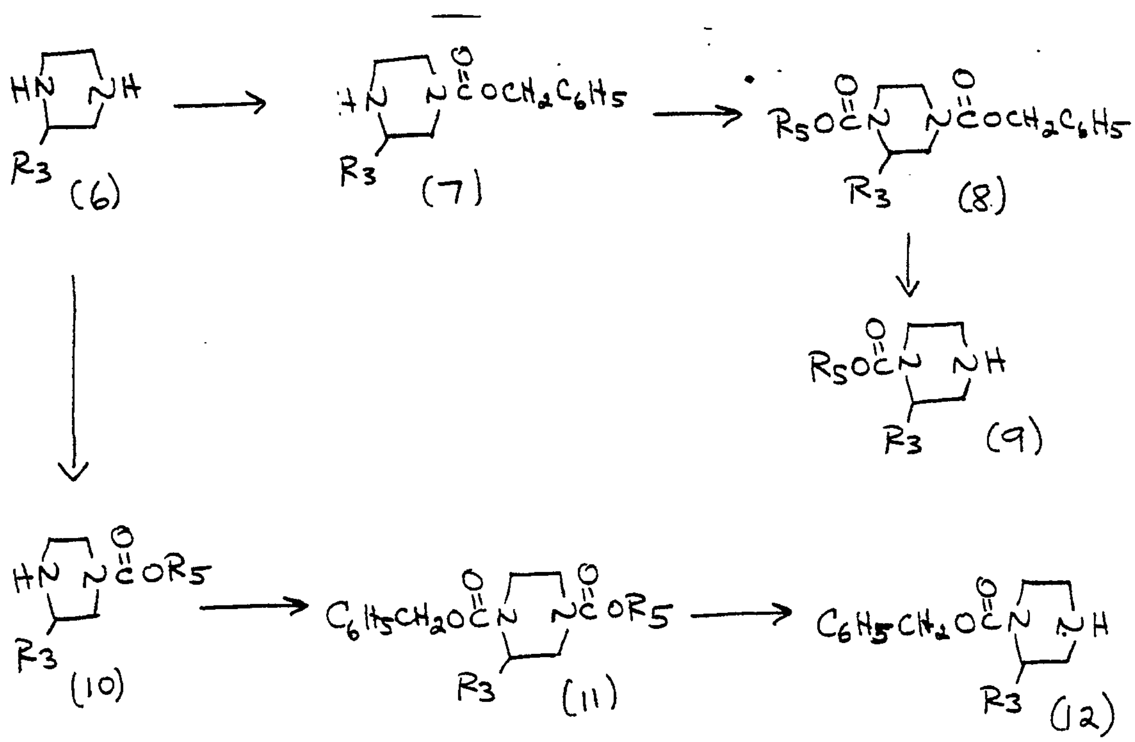

(6) (7) (8) (9) (10) (11) (12)

In this reaction sequence, piperazine (6) is monoprotected, using standard synthetic methods, with a benzyloxycarbonyl (Cbz) or a (loweralkyl)oxycarbonyl, for example t-butyloxycarbonyl (Boc), protecting group to give (7) or (10), respectively. When $R_3$ is not hydrogen, the two nitrogen atoms of the piperazine are not equivalent and separate synthetic routes are needed to obtain (9) and (12). Cbz-piperazine (7) is reacted with a di(loweralkyl)dicarbonate to provide carbamate derivative (8). Hydrogenation, or other standard deprotection methods, removes the Cbz group to give (9, $R_5$ = loweralkyl).

Alternatively, reaction of (6) with a di(loweralkyl)dicarbonate gives (10, $R_5$ = loweralkyl). Compound (10) is further protected with a Cbz group to give carbamate (11). Selective removal of the (loweralkyl)-oxycarbonyl group, using mild acid conditions, provides Cbz-piperazine (12).

In addition to using carbamate protected piperazines wherein the nitrogen protecting group is $R_4OC(O)$- wherein $R_4$ is loweralkyl or benzyl, carbamate protected piperazines wherein $R_4$ may also be cyclopropylmethyl, diisopropylmethyl, 9-fluorenylmethyl, 9-(2-sulfo)fluorenylmethyl, 2-furanylmethyl, 2,2,2-trichloroethyl, 2-bromoethyl, 2-iodoethyl, 2-trimethylsilylethyl, 2-methylthioethyl, 2-methylsulfonylethyl, 2-(p-toluenesulfonyl)ethyl, 2-phosphonioethyl, 1,1-dimethylpropynyl, 1,1-dimethyl-3-(N,N-dimthylcarboxamido)propyl, 1,1-diphenyl-3-(N,N-diethylamino)propyl, 1-methyl-1-(1-adamantyl)ethyl, 1-methyl-1-phenylethyl, 1-methyl-1-(3,5-dimethoxyphenyl)ethyl, 1-methyl-1-(4-biphenyl)ethyl, 1-methyl-1-(p-phenylazophenyl)ethyl, 1,1-dimethyl-2-chloroethyl, 1,1-dimethylbromoethyl, 1,1-dimethyl-2,2,2-trichloroethyl, 1,1-dimethyl2-cyanoethyl, cyclobutyl, 1-methylcyclobutyl, cyclopenyl, cyclohexyl, 1-methylcyclohexyl, 1-adamantyl, isobornyl, allyl, phenyl, 2,4,6-tri-tert-butylphenyl, m-nitrophenyl, 8-quinolyl, N-hydroxypiperidinyl, 4-(1,4-dimthylpiperidinyl), 4,5-diphenyl-3-oxazolinyl-2-one, 2,4,6-trimethylbenzyl, p-methoxybenzyl, 3,5-dimethoxybenzyl, p-decyloxybenzyl, p-nitrobenzyl, o-nitrobenzyl, 3,4-dimethoxy-6-nitrobenzyl, p-bromobenzyl, chlorobenzyl, 2,4-dichlorobenzyl, p-cyanobenzyl, o-(N,N-dimethylcarboxamido)benzyl, m-chloro-p-acyloxybenzyl, p-(phenylazo)-benzyl, 5-benzisoxazolylmethyl, 9-anthrylmethyl, diphenylmethyl, di(2-pyridyl)methyl, 1-methyl-1-(4-pyridyl)-ethyl, isonicotinyl, and the like are also useful for the process of this invention (See T.W. Greene, Protective Groups in Organic Synthesis, pp.223-248, John Wiley & Sons (1981)). By selecting carbamate protecting groups that can be selectively deprotected, one skilled in the art can use the method of Scheme 1 to prepare N-protected (carbamate) piperazine derivatives containing any of the above carbamate N-protecting groups.

Representative of the compounds that can be prepared by the process of the present invention are the following:

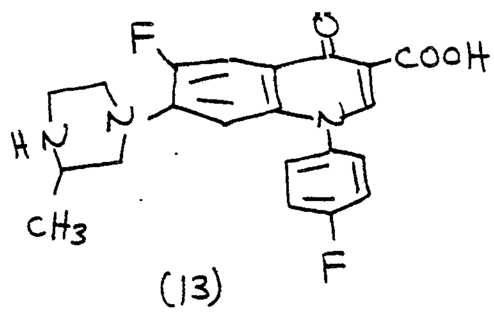

(13)

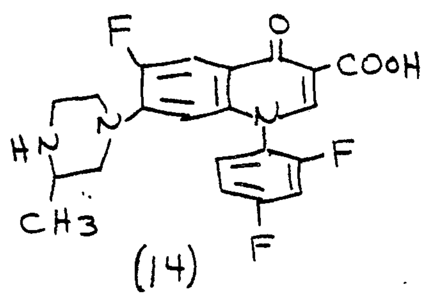

(14)

The term "loweralkyl" as used herein refers to straight or branched chain alkyl radicals containing 1 to 6 carbon atoms.

The term "loweralkenyl" as used herein refers to a $C_2$ to $C_6$ alkyl group having at least one double bond, including, but not limited to vinyl and propenyl.

The term "loweralkynyl" as used herein refers to a $C_2$ to $C_6$ alkyl group having at least one triple bond.

The term "cycloalkyl" as used herein refers to a $C_3$ to $C_7$ carbocyclic group.

The term "hydroxyalkyl" as used herein refers to a loweralkyl radical substituted with one or more hydroxy groups.

The term "alkoxyalkyl" as used herein refers to a loweralkyl radical substituted with one or more alkoxy groups.

The term "alkoxy" as used herein refers to $-OR_8$ wherein $R_8$ is a loweralkyl group, including, but not limited to methoxy, ethoxy and the like.

The term "dialkylamino" as used herein refers to $-NR_9R_{10}$ wherein $R_9$ and $R_{10}$ are independently selected from loweralkyl groups.

The term "(N-protected)(alkyl)amino as used herein refers to $-NR_{11}R_{12}$ wherein $R_{11}$ is an N-protecting group and $R_{12}$ is a loweralkyl group.

The term "(N-protected)amino" as used herein refers to $-NHR_{13}$ wherein $R_{13}$ is an N-protecting group.

The term "alkanoyl" as used herein refers to $-C(O)R_{14}$ wherein $R_{14}$ is a loweralkyl group.

The term "alkanoylamino" as used herein refers to $-NHC(O)R_{15}$ wherein $R_{15}$ is a loweralkyl group.

The term "dialkylaminoalkyl" as used herein refers to a loweralkyl radical substituted with a dialkylamino group.

The term "(N-protected)(alkyl)aminoalkyl" as used herein refers to a loweralkyl radical substituted with an (N-protected)(alkyl)amino group.

The term "(N-protected)aminoalkyl" as used herein refers to a loweralkyl radical substituted with an (N-protected)amino group.

The term "halo" or "halogen" as used herein refers to fluoro, bromo, chloro or iodo.

The term "haloalkyl" as used herein refers to a loweralkyl group in which one or more hydrogens has been replaced by a halogen, including, but not limited to fluoromethyl, trifluoromethyl and the like.

The terms "N-protected", "N-protecting group" or "nitrogen protecting group" as used herein refer to those groups intended to protect a nitrogen atom against undesirable reactions during synthetic procedures and having the formula

$$R_6\,O\overset{\overset{\displaystyle O}{\|}}{C}\,-$$

wherein $R_6$ is loweralkyl or benzyl, including, but not limited to ethoxycarbonyl, tert-butyloxycarbonyl, benzyloxycarbonyl and the like.

The term "carboxy-protecting group" as used herein refers to ester groups commonly employed to block or protect the carboxylic acid functionality while reactions involving other functional sites of the compound are carried out. In addition, a carboxy-protecting group can be hydrolyzed enzymatically to release the biologically active parent acid. Such protected carboxy groups are noted for their ease of cleavage by hydrolytic methods to the corresponding carboxylic acid. Further, such carboxy-protecting groups can be relatively easily cleaved to yield the corresponding free carboxy group. Such carboxy-protecting groups are well known to those skilled in the art, having been extensively used in the protection of carboxy groups in the penicillin and cephalosporin fields, as described in U.S. Patent Nos. 3,840,556 and 3,719,667, the disclosures of which are incorporated herein by reference. Representative protecting groups include, but are not limited to, $C_1$ to $C_8$ alkyl (e.g., methyl, ethyl, tertiary butyl and the like), benzyl and substituted derivatives thereof such as alkoxy- and nitro-benzyl groups, dialkylaminoalkyl (e.g., dimethylaminoethyl and the like), and acyloxyalkyl groups such as pivaloyloxymethyl and propionyloxymethyl.

The foregoing may be understood more fully from the following examples, which are presented for purposes of illustration and are not intended to limit the scope of the invention as defined in the claims.

## Example 1

### 1-t-Butyloxycarbonyl-2-methylpiperazine

A 200 gallon glass-lined reactor was charged with 21 kg of methylene chloride, 20 kg of 2-methylpiperazine and 33.4 kg of sodium bicarbonate, then stirred and cooled to -10°C. It was further charged with 34.1 kg of benzylchloroformate at such a rate that the temperature was maintained at -10°C to -20°C. Upon completion of the addition of the benzylchloroformate, the pressure can and line by which the benzylchlorofromate was added were rinsed with 25 kg of methylene chloride and this was also charged to the reactor. The reaction mixture was then stirred at 10°C ±5°C for one hour.

Solids were removed from the above reaction mixture by centrifugation and the filtrate was charged to a 150 gallon stainless steel reactor. The solids were washed with 100 kg of methylene chloride and then discarded. The filtrate was transfered back to the 200 gallon glass-lined reactor and 50 gallons of tap water was added. The pH of the mixture was adjusted to pH 2-3 using concentrated HCl. The layers were separated and were washed with 40 gallons of methylene chloride and 20 gallons of water respectively.

The combined aqueous phases were charged back to the 200 gallon glass-lined reactor. 40 gallons of methylene chloride was charged to the 200 gallon glass-lined reactor and stirred vigorously. The pH was adjusted to pH 10-11 using 50% aqueous sodium hydroxide solution. The layers were allowed to separate and the aqueous layer was washed with 20 gallons of methylene chloride.

The methylene chloride phases were then combined, dried with 5 kg of magnesium sulfate, filtered and transferred back into a cleaned 200 gallon glass-lined reactor. To the 200 gallon glass-lined reactor was charged 39.2 kg of di-tert-butyldicarbonate, maintaining the temperature below 30°C and stirring for one more hour.

The reaction was quenched by charging 26 gallons of 1.2M sodium hydroxide solution to the reactor

and stirring for 3 hours at ambient temperature. The layers were then allowed to settle and the methylene chloride layer was drained into a 150 gallon stainless steel reactor where it was dried with 5 kg of magnesium sulfate.

The magnesium sulfate was filtered and the filtrate was transferred into the cleaned 200 gallon glass-lined reactor and the solvent was removed in vacuo. Residual solvent (methylene chloride) was distilled and diluted with 10 gallons of methanol, followed by further distillation in vacuo. The residual oil was diluted with 20 gallons of methanol and stored for the next reaction step. The yield was approximately 35 gallons of solution. The product was assayed by thin layer chromatography. A second lot was prepared analogously with the insertion of an additional extraction step for further purification of the 1-tert-butyloxycarbonyl-2-methyl-4-benzyloxycarbonylpiperazine, to yield approximately 38 gallons of solution. This lot was identical to the first lot as assayed.

A 50 gallon glass-lined reactor (set up for hydrogenation) was purged with nitrogen and charged with 2.0 kg of dry 10% palladium on carbon. About one-half of the first lot of 1-tert-butyloxycarbonyl-2-methyl-4-benzyloxycarbonylpiperazine (in methanol) was charged to the 50 gallon reactor (in this case 131 lbs of solution). The reaction was further diluted by the addition of another 656 lbs of methanol to the 50 gallon reactor. The reactor was purged several more times with nitrogen and then hydrogen, and cooling water was then circulated in the reactor jacket. The reactor was pressurized to 50 psi with hydrogen and agitate vigorously for one hour. The reactor was depressurized and then repressurized with hydrogen to 50 psi. The agitation was started again and this procedure of depressurizing and repressurizing continued until the reaction was complete.

At the completion point of the reaction, the reactor was purged twice with nitrogen and the catalyst was filtered from the reaction mixture. The reactor and catalyst were rinsed with methanol. The solvent was removed at 45 ± 5°C (25-28 mm Hg) to yield the title compound. 300 MHz $^1H$ NMR ($CDCl_3$) 4.17 (br m, 1H), 3.78 (br m, 1H), 3.0-2.6 (mm, 6H), 1.48 (s, 9H), 1.21(d, 3H).

## Example 2

### 1-(2,4-Difluorophenyl)-1,4-dihydro-4-oxo-6-fluoro-7-(3-methyl-4-tert-butyloxycarbonyl-piperazin-1-yl)-3-quinoline carboxylic acid

A 50 gallon glass lined reactor was purged with nitrogen and charged with 44.5 kg of dimethylsulfoxide, 2.3 kg of triethylamine, 6 kg of methanol, 5.0 kg of 1-(2,4-difluorophenyl)-6-fluoro-7-chloro-1,4-dihydro-4-oxo-quinoline carboxylic acid (see U.S. Patent No. 4,730,000) and 12 kg of 1-tert-butyloxycarbonyl-2-methylpiperazine. The slurry was stirred and heated to 95 ± 2°C. After 2 hours, 3 kg more of 1-tert-butyloxycarbonyl-2-methylpiperazine and 3 liters of methanol were added. After 4 and 27 hours, respectively 5 kg and 3 kg more of 1-tert-butyloxycarbonyl-2-methylpiperazine were added. After 36 hours, the reaction temperature was lowered to 60°C. To the stirred mixture was added 41 liters of isopropanol that had been preheated to 55°C.

The slurry resulting from the above reaction was cooled to room temperature, filtered and washed with isopropanol. The filter cake was added to 25 liters of 100°C acetic acid and heated to reflux. The slurry was cooled to 19°C, filtered and washed with isopropanol. The solid was dried at 40°C overnight, yielding 3.1 kg of the title compound. m.p. 254-256°C. 300 MHz $^1H$ NMR 8.60 (s, 1H), 8.10 (d, 1H), 7.50 (m, 1H), 7.28-7.15 (m, 3H), 6.21 (d, 1H), 4.33 (br d, 1H), 3.94 (br d, 1H), 3.41-3.18 (m, 4H), 2.91 (m, 1H), 2.72 (m, 1H), 1.46 (s, 9H), 1.30 (d, 3H).

Alternatively, to a solution of 845 mg of 1-(2,4-difluorophenyl)-6-fluoro-7-chloro-1,4-dihydro-4-oxo-3-quinoline carboxylic acid in 6 mL of 1-methyl-2-oxo-pyrrolidine at 90°C under nitrogen was added 2.0 gm of 1-tert-butyloxycarbonyl-2-methyl-piperazine. After 2 days, the mixture was cooled and 1 mL of acetic acid was added.

50 mL of water was then added and a solid residue was filtered from the mixture. The residue was washed with water and dried. The solid was then suspended in 20 mL of ethanol and boiled for 4-5 hours. After cooling, the suspension was filtered, yielding 495 mg (40%) of the title compound.

## Example 3

1-(2,4-Difluorophenyl)-1,4-dihydro-4-oxo-6-fluoro-7-(3-methyl-4-ethoxycarbonyl-piperazin-1-yl)-3-quinoline carboxylic acid

To a solution of 310 mg of 1-(2,4-difluorophenyl)-6-fluoro-7-chloro-1,4-dihydro-4-oxo-quinoline carboxylic acid (see U.S. Patent No. 4,730,000) in 3 mL of N-methylpyrrolidone was added 530 mg of 1-ethoxycarbonyl-2-methylpiperazine. The solution was heated at 80 °C for 3 days and then 60 mL of water was added. After removing the insoluble material, 6 mL of 1N HCl was added and the resulting precipitate was filtered. The filtered solid was washed with water and ether to provide 130 mg of a solid, which was recrystallized from ethanol to provide the title compound, m.p. 229 °C.

Example 4

6-Fluoro-1-(2,4-difluorophenyl)-1-4-dihydro-7-(3-methylpiperazin-1-yl)-4-oxo-3-quinoline-carboxylic acid hydrochloride

A 30 gallon glass-lined reactor was charged with 12 liters of acetic acid and 3.1 kg of 1-(2,4-difluorphenyl)-1,4-dihydro-4-oxo-6-fluoro-7-(3-methyl-4-tert-butyloxycarbonylpiperazinyl)-3-quinoline carboxylic acid and 1.1 liters of hydrochloric acid. The mixture was heated at 75-80 °C and then 27 liters of distilled water was added.

The solution was filtered with the aid of nitrogen pressure through a filter cartridge into a 50 gallon glass-lined reactor. The solution was then heated to about 90-95 °C and 2.5 liters of hydrochloric acid was added. The mixture was cooled to 18 °C over about 2 hours. The solid was filtered and rinsed with 15 liters of 2N hydrochloric acid and the solid was dried under vacuum. The total yield was 2553 gm of the title compound. m.p. 290 °C (dec.). 300 MHz $^1H$ NMR (DMSO-d6) 9.30 (2H), 8.76 (s, 1H), 8.00 (d, 1H), 7.92 (dtrp, 1H, J = 5.9, 8.8 Hz), 7.63 (m, 1H), 7.42 (m, 1H), 6.41 (dd, 1H, J = 7.2 Hz), 3.65-3.00 (m, 7H), 1.30 (d, 3H).

Alternatively, to a solution of 140 mg of the product of Example 2 in 1 mL of trifluoroacetic acid was added 2 mL of 3N HCl. This was stired for 1 hour at room temperature and then evaporated to dryness, yielding a yellowish-green solid. 15 mL of $CH_3CN$ was added, giving a yellowish solution. After boiling for 2 to 3 minutes, a white solid appeared and the solution became colorless. The solution was cooled and filtered, yielding 118 mg (95%) of the title compound.

A further alternative procedure involved heating 340 mg of the product of Example 3 in 12 mL of 6N HCl at 110 °C for 4 days. Evaporation of the solvent provided the desired product.

It will be understood that various changes and modifications can be made in the details of the procedure to adapt it to various conditions without departing from the spirit of the invention, especially as defined in the appended claims.

**Claims**

1. A process for preparing a 1,4-dihydro-6-fluoro-7-(piperazin-1-yl)-4-oxo-3-quinoline carboxylic acid derivative of the formula:

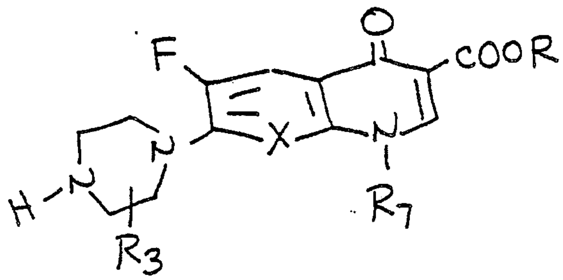

wherein R is hydrogen or a carboxy-protecting group; $R_7$ is loweralkyl, loweralkenyl, $C_3$ to $C_7$ cycloalkyl, haloalkyl or

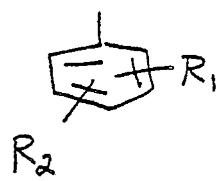

wherein $R_1$ is halogen and $R_2$ is hydrogen or halogen; $R_3$ is hydrogen, loweralkyl, haloalkyl, loweralkenyl, loweralkynyl, $C_3$ to $C_7$ cycloalkyl, hydroxyalkyl, alkoxyalkyl, (N-protected)(alkyl)aminoalkyl, dialkylaminoalkyl, (N-protected)aminoalkyl, hydroxy, alkoxy, halogen, alkanoyl, alkanoylamino, (N-protected)amino, (N-protected)(alkyl)amino or dialkylamino; and X is CH, CCl, CF or N comprising:

a) reacting a carbamate protected piperazine derivative of the formula

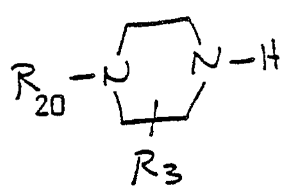

wherein $R_3$ is hydrogen, loweralkyl, haloalkyl, loweralkenyl, loweralkynyl, $C_3$ tc $C_7$ cycloalkyl, hydroxyalkyl, alkoxyalkyl, (N-protected)(alkyl)aminoalkyl, dialkylaminoalkyl, (N-protected)aminoalkyl, hydroxy, alkoxy, halogen, alkanoyl, alkanoylamino, (N-protected)amino, (N-protected)(alkyl)amino or dialkylamino; and $R_{20}$-N taken together form carbamate group with a compound of the formula

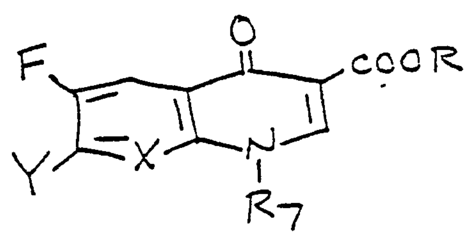

wherein R is hydrogen or a carboxy-protecting group; $R_7$ is loweralkyl, loweralkenyl, $C_3$ to $C_7$ cycloalkyl, haloalkyl or

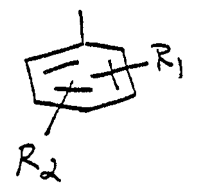

wherein $R_1$ is halogen and $R_2$ is hydrogen or halogen; X is CH, CCl, CF or N; and Y is F or Cl; followed by

b) deprotection of the piperazine nitrogen.

2. The process of Claim 1 wherein R is hydrogen, $R_7$ is difluorophenyl, $R_3$ is methyl, $R_{20}$ is tert-butyloxycarbonyl or benzyloxycarbonyl, X is CH and Y is Cl.

3. The process of Claim 1 wherein R is hydrogen, $R_7$ is o,p-difluorophenyl, $R_3$ is 3-methyl, $R_{20}$ is tert-butyloxycarbonyl, X is CH and Y is Cl.

4. A process for preparing a 1,4-dihydro-6-fluoro-1-(halo-substituted phenyl)-7-piperazin-1-yl)-4-oxo-3-quinoline carboxylio acid of the formula:

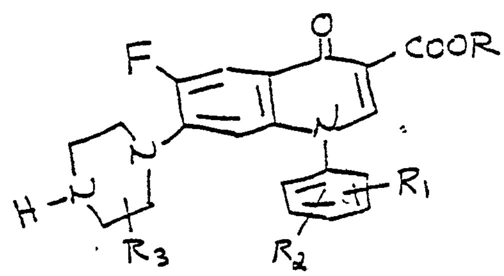

wherein R is hydrogen or a carboxy-protecting group; $R_1$ is halogen; $R_2$ is hydrogen or halogen; and $R_3$ is hydrogen, loweralkyl, haloalkyl, loweralkenyl, loweralkynyl, $C_3$ to $C_7$ cycloalkyl, hydroxyalkyl, alkoxyalkyl, (N-protected)(alkyl)aminoalkyl, dialkylaminoalkyl, (N-protected)aminoalkyl, hydroxy, alkoxy, halogen, alkanoyl, alkanoylamino, (N-protected)amino, (N-protected)(alkyl)amino or dialkylamino, comprising:

a) reacting an N-protected piperazine derivative of the formula

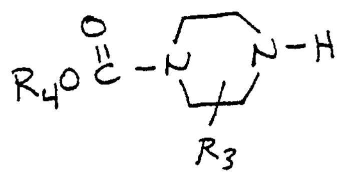

wherein $R_3$ is hydrogen, loweralkyl, haloalkyl, loweralkenyl, loweralkynyl, $C_3$ to $C_7$ cycloalkyl, hydroxyalkyl, alkoxyalkyl, (N-protected)(alkyl)aminoalkyl, dialkylaminoalkyl, (N-protected)aminoalkyl, hydroxy, alkoxy, halogen, alkanoyl, alkanoylamino, (N-protected)amino, (N-protected)(alkyl)amino or dialkylamino and $R_4$ is loweralkyl or benzyl with a compound of the formula

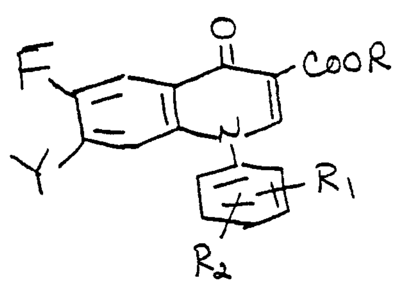

wherein R is hydrogen or a carboxy-protecting group, $R_1$ is halogen, $R_2$ is hydrogen and Y is Cl or F; followed by

b) deprotection of the piperazine nitrogen.

5. The process of Claim 4 wherein R is hydrogen, $R_1$ and $R_2$ are fluoro, $R_3$ is methyl, $R_4$ is tert-butyl or benzyl and Y is Cl.

6. The process of Claim 4 wherein R is hydrogen, $R_1$ is o-fluoro, $R_2$ is p-fluoro, $R_3$ is 3-methyl, $R_4$ is tert-butyl and Y is Cl.

7. The process of Claim 4 wherein $R_4$ is loweralkyl or benzyl and the piperazine nitrogen is deprotected by treatment with acid.

8. The process of Claim 4 wherein $R_4$ is benzyl and the piperazine nitrogen is deprotected by hydrogenolysis.

9. A process for preparing 6-fluoro-1-(2,4-difluorphenyl)-1,4-dihydro-7-(3-methylpiperazin-1-yl)-4-oxo-3-quinoline carboxylic acid, comprising reacting 2-methyl-1-tert-butyloxycarbonylpiperazine with 7-chloro-6-fluoro-1-(2,4-difluorophenyl)-1,4-dihydro-4-oxo-3-quinoline carboxylic acid, followed by deprotection of the piperazine nitrogen.

10 The process of Claim 9 wherein the piperazine nitrogen is deprotected by treatment with acid.

European Patent Office

# EUROPEAN SEARCH REPORT

Application Number

EP 89 11 2960

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 131 839 (ABBOTT LABORATORIES) * Claim 19; page 13, line 34 - page 14, line 19; page 9, reaction scheme * & US - A - 47 30000 (Cat. D) | 1 | C 07 D 215/56<br>C 07 D 401/04<br>C 07 D 471/04 //<br>(C 07 D 471/04<br>C 07 D 221:00<br>C 07 D 221:00 ) |
| A | EP-A-0 009 425 (LABORATOIRE R. BELLON) * examples 7,8 * | 1 | |
| | | | TECHNICAL FIELDS SEARCHED (Int. Cl.4) |
| | | | C 07 D 215/00<br>C 07 D 401/00<br>C 07 D 471/00 |

The present search report has been drawn up for all claims

| Place of search | Date of completion of the search | Examiner |
|---|---|---|
| BERLIN | 18-10-1989 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)